# EUROPEAN PATENT APPLICATION

(11) **EP 4 621 059 A2**
(43) Date of publication of application: **24.09.2025**
(21) Application number: 25163555.3
(22) Date of filing: 13.03.2025
(51) Int. Cl.: C12P 5/02, C12M 1/107, B01F 23/231, B01F 23/237, C12M 1/00

(54) **PROCESS AND APPARATUS FOR THE PRODUCTION OF BIOGAS IN ANAEROBIC DIGESTION PLANTS**

(30) Priority: 19.03.2024 IT 202400006055
(71) Applicant: SOL S.p.A., 20900 Monza (MB) (IT); ALCHIMIA S.r.l., 27027 Gropello Cairoli (PV) (IT)
(72) Inventor: CASTIGLIONI, Alberto, 27027 Gropello Cairoli (PV) (IT); BECCALLI, Massimo, 20900 Monza (MB) (IT)
(74) Representative: Trupiano, Federica

(57) **Abstract**

The present invention relates to a process, and related equipment, for increasing the production of bio-methane in biogas in anaerobic digestion plants, that takes advantage of injecting carbon dioxide in bubble form, either alone or in mixture with other gases, into the biomass. The present invention also relates to a control and/or measurement system of the digestate pH during the process of the invention.

## Description

### Summary of the invention

The present invention relates to a process, and related equipment, for increasing the production of bio-methane in biogas in anaerobic digestion plants, that takes advantage of injecting carbon dioxide in bubble form, either alone or in mixture with other gases, into the biomass. The present invention also relates to a control and/or measurement system of the digestate pH during the process of the invention.

### Technical background

Anaerobic digestion (AD) of biomass is a biological process that takes place in the absence of oxygen and is aimed at producing biogas consisting mainly of methane (CH₄) and carbon dioxide (CO₂) and also containing a small amount of hydrogen and traces of other compounds such as, for example, hydrogen sulfide. The residual biomass, after AD treatment, is called digestate.

Inside today's plants, the process of anaerobic digestion allows biomass to be transformed into an important energy resource, thanks to the possible use of biogas as a fuel, either directly or as a methane-only component (bio-methane) after its *upgrading* by CO₂ removal. Digestate, on the other hand, is considered a valuable soil conditioner for fertilizing agricultural land.

What happens inside AD plants is quite similar to what happens in the gastrointestinal system of ruminants, where the gas produced during the physiological processes of digestion constitutes a natural waste, i.e. gaseous excretions.

The percentage of methane produced in AD processes varies depending on the type of starting organic substance and process conditions, normally such percentage is between about 40% and 80% of the biogas produced.

From a biological point of view, for the process to take place, the action of different microorganisms capable of transforming organic substance into intermediate compounds, such as acetic acid (CH₃COOH), carbon dioxide (CO₂) and hydrogen (H₂), is required; said compounds are then used as substrates by methanogenic bacteria that conclude the process by producing methane (CH₄). There are basically four steps in the process: hydrolysis, acidogenesis, acetogenesis and methanogenesis; different families of microorganisms are needed to carry out all said process steps. Furthermore, it is essential for successful AD that the conditions of the reaction environment of the anaerobic digestion plant are maintained at optimal values. Said conditions are mainly represented by pH, which, normally, is maintained between values of 7 to 8, and temperature, normally between 35°C and 55°C depending on the main types of microorganisms involved.

The residence time of the organic substance, which will be transformed into digestate and biogas inside an AD plant, varies depending on the amount of biomass to be processed, the type of biomass, and the operating temperature. For example, in the case of digestion carried out with mesophilic bacteria, which is less expensive and currently more widely used because it requires lower temperatures (lower absorption of heat energy), the residence time is generally between 15 and 60 days. However, thermophilic digestion, which, on the other hand, has a higher cost because it requires more energy and is more critical than the similar mesophilic process, has shorter residence times, generally ranging from 7 to 30 days. A direct consequence of the timing of the process is the fact that the volumes of an AD plant are normally large and the diameters of the digestion reactors are in the order of tens of meters.

An anaerobic digestion plant, or digester, in addition to the raw material storage and preparation section and product storage and treatment section, basically consists, as far as the digestion section is concerned, of one or more reactors fed continuously or in *batches* in series or in parallel, which are equipped with biogas capture systems.

The most common reactors are those that are continuously agitated with scheduled feeding; they have mechanical or hydraulic devices adapted to mix the material contained inside them (the biomass) in order to promote contact between bacteria and the entire loaded substrate, thus avoiding the presence of "dead" zones. Furthermore, the ability to keep the substrate moving allows for uniform temperature in all areas of the reactor as well as facilitating biogas release and avoiding sludge sedimentation and the formation of surface layers of unmixed material. Since the solids content inside the digester is normally between 2% and 20% (with the exception of so-called "dry" digesters, which can reach up to 35% solids content), the most common mixing technologies take advantage of either mechanical agitators and mixers and/or systems for extracting and recirculating material inside the reactor itself.

In recent years, there has also been a widespread need to provide for the separation of the CO₂ component from biogas, as a result of legislative measures that have incentivized the production of methane of biological origin. However, the gaseous CO₂ produced and recovered in this type of AD plants is not easily reused industrially as it is more expensive than that from other types of plants.

In the state of the art, there are some documents, see for example WO2008002538, that propose the generic possibility of using carbon dioxide, derived from the combustion of fossil fuels, to increase methane production inside anaerobic digesters.

However, to date, no real applications of such possibility are known, as the actual technical modes regarding how to feed said gas uniformly into the biomass remain problematic, especially in relation to the normally high concentration of suspended solids desirable in anaerobic digestion systems to minimize the high volumes that come into play.

Therefore, there is still a great need to have a process for increasing bio-methane production in biogas that would allow for a significant increase in bio-methane production under the same conditions. Said process must also be cost-effective and applicable to all possible different conditions of use of the digester itself (such as, for example, temperature, type of bacteria, maturation time, percentage amount of solid constituting biomass, and so on).

### Objects of the invention

An object of the present invention is to provide a process for producing biogas, inside an apparatus for the anaerobic digestion of biomass, or anaerobic digester, that is more efficient than processes known to date in the state of the art and that allows a greater amount of biogas, with corresponding fraction of bio-methane, to be obtained.

Another object of the present invention is to provide an apparatus that allows to carry out said process for producing biogas and the corresponding bio-methane fraction.

Further object of the present invention is to provide a system for analysis and control of said biogas production process that allows continuous adjustment of its process parameters.

Further object of the invention is to provide a biogas production apparatus that allows agile and simple maintenance and cleaning operations.

These and other objects are achieved by the subject matter of the present invention, which concerns a process, and related apparatus, for increasing bio-methane production in the biogas in anaerobic digesters.

### Description of the figures

Figure 1: histogram of methane production (L) over time (days) in a lab-scale digester in which CO₂ was insufflated (gray bars) compared with methane production (L) in a lab-scale digester in which CO₂ was not insufflated (white bars);
Figure 2: graph of methane production (L) over time (days) in a lab-scale digester in which CO₂ was insufflated (filled indicators) compared with methane production (L) in a lab-scale digester in which CO₂ was not insufflated (empty indicators);
Figure 3: front view of a possible embodiment of the digester according to the invention;
Figure 4A: plan view of a possible embodiment of the digester according to the invention;
Figure 4B: detail view of the recirculation pipeline in the possible embodiment of the digester shown in Figure 4A;
Figure 5A: possible embodiment of the microbubble generating device according to the invention;
Figure 5B: cross-sectional view of the possible embodiment of the microbubble generating device according to the invention, shown in Figure 5A;
Figure 5C: longitudinal sectional view of the possible embodiment of the microbubble generating device according to the invention, shown in Figure 5A.

### Description of the invention

Object of the present invention is a process for producing biogas richer in the bio-methane fraction inside an anaerobic digestion (AD) process involving injecting, into the biomass, a gas mixture in the form of bubbles.

Said gas mixture may consist of carbon dioxide alone (100% CO₂ either pure or, normally, industrial grade, as known to the technician skilled in the art) or comprise, in addition to it, other gases (in small percentage, for example, less than 5% by volume to the total volume of the mixture) such as, for example, but not limited to, methane, nitrogen and/or hydrogen, oxygen, either alone or in mixture with each other.

Anaerobic digestion plants of biomass, or anaerobic digesters, that can be used in the present invention can be of any type known to the skilled in the art and use any biomass matrix. Preferably, these are wet anaerobic digesters with cylindrical digesters with vertical axis or vertical walls having a truncated ellipsoidal section.

The present invention is applicable in the field of biomass treatment of any origin, whether from organic waste, especially from biological sewage sludge and/or organic fraction of municipal solid waste, animal effluents, or from industrial by-products specifically designed to be treated in AD plants. For example, anaerobic digestion that can be used according to the present invention can be carried out by having agricultural products, by-products of the agro-industrial industry, animal effluents (manure, slurry of bovine or porcine origin, poultry manure), sludge from biological sewage, organic waste, such as the organic fraction of municipal solid waste from separate collection or mechanical separation, organic waste from the production or use of agro-food products, and animal by-products as feedstock of the process.

The anaerobic digestion process concerned by the present invention can be carried out either under mesophilic conditions, and in particular at a temperature between 33°C and 45°C, preferably between 37°C and 40°C, or under thermophilic conditions, and in particular at a temperature between 50°C and 57°C, preferably between 53°C and 55°C.

As previously described, object of the invention is to improve bio-methane production by significant increase of biogas obtained from biomass by anaerobic treatment. Said improvement occurs, from an operational point of view, both in terms of reducing the treatment time for the same amount of biogas produced, and in terms of increasing the bio-methane component inside the biogas for the same feed schedule, treatment time, and AD plant volumes. Said improvement thus allows, for the same amount of bio-methane produced, a reduction in the overall opex (operating expense, i.e., the cost required to manage a product, business or system) and capex (capital expense, i.e., the cost to develop or provide durable assets for the product or system) costs.

Thus, the process according to the present invention is capable of improving the biogas production quantitatively and qualitatively, being able to produce biogas that contains a higher percentage of bio-methane, which is its most valuable energy component.

CO₂ used in the present invention can be of different origins. In particular, it can be derived either from fossil sources, such as from the combustion of hydrocarbons, or be obtained by *upgrading* biogas produced by anaerobic digestion, that is, by separation of the bio-methane fraction from biogas.

According to a preferred and particularly advantageous aspect of the present invention, CO₂ that is fed into the apparatus for the anaerobic digestion of biomass, or digester, in the form of bubbles, either alone or in a mixture with other gases, is that produced by the anaerobic digestion process itself and recovered from the biogas leaving the digester, after separation from the methane fraction.

Advantageously, the recovery and reuse of CO₂ produced by the anaerobic digestion process allows increased production of biogas and bio-methane, as a result of its return into the digestion process, by using an immediately available and essentially cost-free resource. Furthermore, recovery and reuse of CO₂ produced by the anaerobic digestion process allow the environmental sustainability of the process and apparatus to be improved, significantly reducing the amount of carbon dioxide that is released into the atmosphere.

In embodiments, CO₂ produced by the anaerobic digestion process and reused in the same process can be in the form of a waste gas commonly referred to as "*off gas",* that is, a gas mixture that has very high concentrations of CO₂, with values as low as 98% but typically being 99-99.5% volume/volume, with residual gas consisting of methane and traces (ppm) of other gases (for example, H₂S).

According to another preferred aspect of the present invention, the gas mixture comprising CO₂, alone or in mixture with other gases, is fed into the digester in the form of bubbles of controlled size. Preferably, said bubbles are micrometric in size, for example they have an average diameter between 50 µm and 2,000 µm, preferably between 100 µm and 1,000 µm. For example, said bubbles may be < 500 µm in diameter, preferably between 100 µm and 200 µm.

Injecting the gas mixture in the form of bubbles into the digester, preferably in the form of microbubbles of the previously referred size, can be continuous or discontinuous.

According to a preferred aspect of the invention, the injection pressure of the gas containing carbon dioxide according to the present invention ranges from 0.1 bar to 5 bar, preferably from 0.8 bar to 3 bar, even more preferably it is 2 bar.

According to the process of the present invention, the gas containing CO₂ is injected by injecting it into a digestate recirculation circuit 10, as will be better described below.

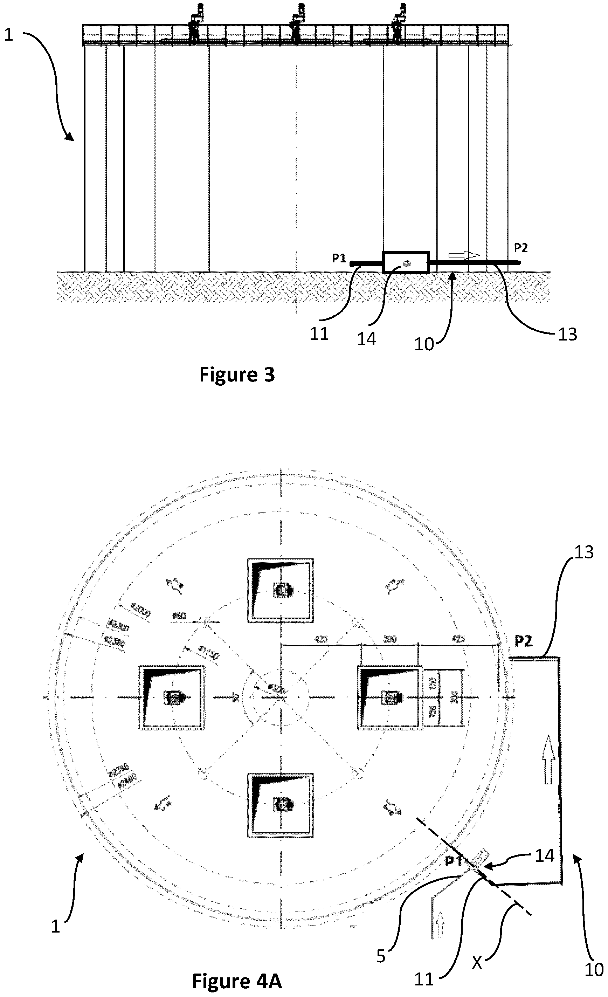

According to a preferred aspect, the apparatus 1 for anaerobic digestion of biomass, or digester 1, shown for illustrative and non-limiting purpose in Figures 3 and 4A, comprises a main body, or closed basin, where the biomass is contained.

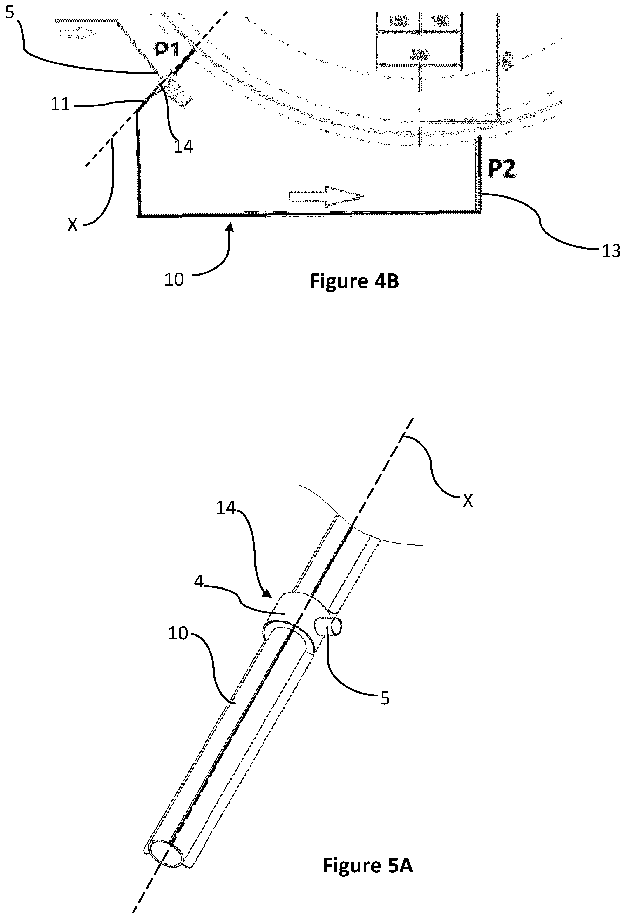

Furthermore, the digester 1 comprises a recirculation circuit 10 of the biomass contained in the digester, as shown in Figures 3, 4A and 4B, comprising an suction branch 11 for such biomass, which is inserted into the digester 1 at a suction position P1 preferably located at the base of the digester 1, and at least one return branch 13 that is inserted into the digester 1 at a return position P2 preferably located at the base of the digester 1, wherein said return position P2 is distinct and away from that position P1.

As shown in the attached Figures 3 - 4B, the recirculation circuit 10 extends between the suction branch 11 inserted into the digester 1 at the one suction position P1, and the return branch 13 inserted into the digester 1 at the one return position P2.

The digestate is thus collected by the suction branch 11 from inside the digester 1 and is re-injected into the digester 1 through the return branch 13.

The digestate recirculation circuit 10 further comprises a recirculation pump, or similar means for suctioning the digestate from inside the digester 1 through the suction branch 11, to the return branch 13 that terminates with a re-injection inlet or nozzle suitably shaped according to the known art, suitable for re-injecting the digestate taken through the suction branch 11 at the position P1 into the digester 1, through the return branch 13.

For example, the pump may be a positive displacement pump of lobe or single screw type, or centrifugal type.

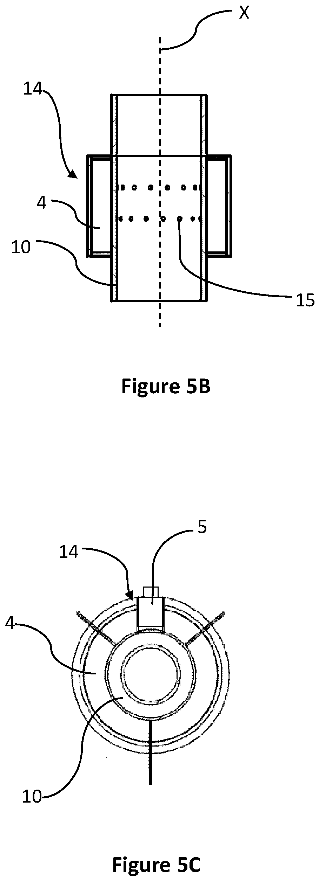

With particular reference to Figures 4B and 5A - 5C, it should be noted that the digester 1 includes a microbubble generating device 14.

The microbubble generating device 14 enables the generation of bubbles of the gas mixture comprising CO₂ according to the present invention, of controlled size, preferably micrometric in size. The bubbles generated by the microbubble generating device 14 ("microbubbles"), for example, may have an average diameter between 50 µm and 2,000 µm, preferably between 100 µm and 1,000 µm. For example, said bubbles may be < 500 µm in diameter, preferably between 100 µm and 200 µm.

The microbubble generating device 14 can be made according to different embodiments.

For example, in a possible embodiment shown in Figures 5A - 5C, the microbubble generating device 14 comprises a channel 4 of preferably annular, more preferably hollow tubular, or toroidal shape, which comprises a plurality of microholes 15 at its inner surface.

In a possible embodiment, the microholes have a diameter between 0.5 mm and 5 mm, preferably equal to 1 mm.

The channel 4 of the microbubble generating device 14 is in fluidic connection with the recirculation circuit 10. In particular, the channel 4 of the microbubble generating device 14 is in fluidic connection with the inside of the recirculation circuit 10.

With reference to detail Figures 5A - 5C, CO₂ is injected into the recirculation circuit 10 through the microbubble generating device 14 which comprises an injection inlet 5 in connection with the gas source comprising CO₂.

In embodiments, the source of gas comprising CO₂ is the digester 1. In other words, in embodiments, the gas mixture comprising CO₂ obtained from anaerobic digestion inside the digester 1 can be collected from the digester 1, through appropriate means for collecting the gas mixture comprising CO₂; the gas mixture comprising CO₂ collected from the digester 1 can be fed again into the digestate passing through the recirculation circuit 10, via the microbubble generating device 14.

In embodiments, means for collecting the gas mixture comprising CO₂ comprise means for purifying CO₂. Advantageously, in this case, a mixture comprising CO₂ only is fed again into the digestate passing through the recirculation circuit 10, via the microbubble generating device 14.

In other words, the digester produces biogas, which is basically a mixture of methane (bio-methane) and CO₂. Biogas, should it be used for bio-methane production, generally undergoes purification (upgrading) according to different methodologies. Obtaining bio-methane from the purification process involves the separation of CO₂ and, normally, the expulsion of this CO₂ into the atmosphere at a high concentration (98 - 99%). According to a preferred aspect of the present invention, it is possible to recover this highly concentrated CO₂ to be re-injected into the digester. In this sense, then, CO₂ that would be injected is CO₂ produced by the digester itself (or by another or by a mix of digesters in the case of multiple digesters, which is the most frequent case in a plant).

According to the present invention, the purification (upgrading) process, mentioned above, is towards bio-methane, whereas CO₂ is a by-product obtained during such purification (upgrading) process.

The injection inlet 5 injects gas containing CO₂ into the channel 4 of the microbubble generating device 14, so that the gas containing CO₂ is subsequently injected into the recirculation circuit 10 through the microholes 15 of the channel 4, in the form of microbubbles.

The channel 4 of the microbubble generating device 14 is placed in fluidic connection with the recirculation circuit 10 and preferably with the inside of the recirculation circuit 10 by means of the microholes 15, which place the channel 4 of the microbubble generating device 14 in fluidic connection with the inside of the recirculation circuit 10.

In particular, as shown in Figure 5B, in a preferred embodiment, the microholes 15 are made on the inner surface of the channel 4 and place the channel 4 of the microbubble generating device 14 in fluidic connection with the inside of the recirculation circuit 10.

In a preferred embodiment, the channel 4 of the microbubble generating device 14 has an annular, preferably hollow tubular or toroidal shape, and extends coaxially with the recirculation channel 10 along a longitudinal axis X which is coaxial to at least one section of the recirculation circuit 10.

In a possible embodiment shown for illustrative and non-limiting purposes in Figures 5A - 5C, the channel 4 of the microbubble generating device 14 extends along at least one portion of the recirculation channel 10, coaxially with the recirculation channel 10.

In an alternative embodiment not shown in the attached figures, the channel 4 of the microbubble generating device 14 extends along the full length of the recirculation channel 10, coaxially to the recirculation channel 10.

Preferably, the injection inlet 5 of the microbubble generating device 14 is placed at the suction branch 11 of the recirculation circuit 10 and is inserted into the channel 4 of the generating device 14 along a direction transverse to the longitudinal axis of the channel 4.

Therefore, injection the gas containing CO₂ can occur along the recirculation circuit 10 of the liquid consisting of the biomass (digestate) in the digester 1. In a possible alternative embodiment not shown in the attached figures, the microbubble generating device 14 comprises a candle diffuser with a porous stone instead of the micro-perforated annular channel 4, or similar devices, for example, micro-perforated membranes, plastic/sintered material diffusers, or the like, also directly installed inside the digester.

In particular, the microbubble generating device 14 enables the production of bubbles of gases containing CO₂ of micrometer size as previously described, between 50 µm and 2,000 µm, preferably between 100 µm and 1,000 µm, due to the presence of the plurality of microholes 15 as described above.

In a possible embodiment, the microbubble generating device 14 comprises a channel 4 that has a length between 5 cm and 50 cm and that can generally affect only a portion of the recirculation circuit 10 or the entire length of it.

Furthermore, the channel 4 may have a variable number of microholes 15, which have a diameter between 0.5 mm and 5 mm.

As discussed above with reference to Figures 3 - 4B, the recirculation circuit 10 extends between the suction branch 11 inserted into the digester 1 at the one suction position P1, and the return branch 13 inserted into the digester 1 at the one return position P2. Advantageously, the gas mixture comprising CO₂ (or CO₂ alone) is diffused inside the digestate, in the form of bubbles, as the digestate passes through the recirculation circuit 10.

Preferably, the digestate enriched with the gas mixture comprising CO₂ is re-injected from the bottom of the digester 1, to allow the CO₂ to move up the entire digestate column in the digester 1. The suction of the digestate from the bottom of the digester 1 further ensures that there is a wide static head for the recirculation pump and allows recirculation under all conditions, even when the digester 1 contains digestate for half its volume. Advantageously, the reciprocal suction P1 and return P2 positions can be spaced to avoid a short-circuit effect and avoid taking back the digestate that has just been re-injected into the digester 1. In embodiments, the suction P1 and return P2 positions can be coplanar, preferably placed at the base of the digester 1, for example, at a distance of 45° to 180°, preferably 120° to 180° in a digester having a circular plan.

It should be noted that the injection of CO₂ in the form of microbubbles causes an increase in the production of biogas and thus bio-methane compared to the insufflation of such gas by other methods of the known art, such as by injecting a continuous stream of CO₂.

Indeed, CO₂ microbubbles are present in the insufflated gas with a very high surface area/volume ratio which results in a high transport coefficient of CO₂ mass from the gas phase (microbubbles) to the liquid phase (biomass in the digester). Such phenomenon results in greater efficiency of CO₂ utilization by the biomass under digestion. Furthermore, injection from the bottom of the digester 1 (feasible, for example, when the suction P1 and return P2 positions are placed at the base of the digester 1) allows adequate reaction time between the CO₂ injected and the biomass before this gas can reach the top of the digester 1.

In a possible embodiment, the recirculation circuit 10 comprises adjusting and measuring means such as valves, thermometers, pressure gauges, pH-meters etc.

Furthermore, the recirculation circuit may comprise inside it, preferably upstream of the pump, plate or tube-in-tube type heat exchangers for adjusting and maintaining the digester temperature.

According to an alternative embodiment, injection of gas containing CO₂ can also occur through the feed line of the digester 1. In this case, the digestate (or biomass) is either in the form of a liquid suspension or water is added thereto to achieve the suspended state of the solid fraction in the liquid.

The line can be equipped with valves, matrix pretreatment systems such as hydrolysis systems, sonication systems, crushers or implosion systems, as well as necessary measuring instruments such as thermometers, manometers, pH-meters and pumps.

According to a preferred aspect of the present invention, the CO₂ amount injected daily into the digester ranges from 0.1 L/day to 2.2 L/day CO₂, either alone or in admixture with other gases, per L of digestate in the digester. Preferably, the CO₂ amount injected daily, alone or in admixture with other gases, is between 0.2 L/day and 1.5 L/day, preferably between 0.3 L/day and 1 L/day; for example, 0.2 L/day and 1 L/day per L of digestate.

Further object of the present invention is a system for analyzing and controlling the process for biogas production as previously described that enables the adjustment of its process parameters.

In particular, object of the present invention is a control and/or measurement system of the digestate pH during the process of the invention, equipped with a system that allows the automatic activation and/or interruption of CO₂ insufflation upon reaching preset threshold values and/or preset variations in said pH values.

According to a preferred aspect of the present invention, adjusting the amount of gas containing CO₂ injected into the digester, as well as the injection mode (continuous or discontinuous), is actually determined by the pH value of the digestate, which can be measured, as previously mentioned, by means of a pH-meter that can be placed in line with the digestate injecting and/or recirculating system itself or that is already present in the instrumentation installed on the AD plant, or by collecting aliquots of digestate.

In particular, upon the application of an experimental protocol which allowed the best operating conditions of the process of the present invention to be established, it was verified that injecting gas containing CO₂ in the form of bubbles, preferably microbubbles with an average size between 50 µm and 1,000 µm, brings the best effects in terms of effectiveness in biogas production, when a decrease in the pH value of the digestate between 0.1 and 1 pH point, preferably between 0.2 and 0.4 pH points, is measured.

Thus, the control and/or measurement system of the digestate pH during the process of the present invention will allow the insufflation of CO₂ to be interrupted following the decrease in the value of digestate pH from 0.1 to 1 pH point, preferably from 0.2 to 0.4 pH points, and the insufflation of CO₂ to be activated, following the increase in the value of digestate pH from 0.1 to 1 pH point, preferably from 0.2 to 0.4 pH points.

For illustrative and non-limiting purposes, for example, the following values can be identified: the addition of 0.5 L/day of gaseous CO₂ under standard conditions (i.e., at 1 bar pressure and 15°C) per liter of digestate results in a decrease of about 0.3 pH points compared to a digestate into which no CO₂ is injected. Said decrease of pH point value is considered to be the one desired and to be maintained throughout the digestion process to determine the increase in biogas production and, in particular, the increase in its fraction in bio-methane.

Again for illustrative, and not limiting, numerical purposes, it can be set forth that if the initial pH value of the digestate is, for example, pH 7.9, CO₂ will be injected in the form of microbubbles, preferably microbubbles between 50 µm and 2,000 µm in size, preferably between 100 µm and 1,000 µm, until a pH value of 7.6 is reached. The addition of gas will be kept by continuing to monitor the pH value, which, should it drop further, for example, to a pH value of 7.4, said value will show a threshold at which said gas addition will be stopped. Monitoring the pH value will be kept so that gas injection can be restarted once the pH value reaches pH 7.6 once again.

Therefore, in embodiments, the process of the invention may further comprise a step of interrupting the injection of the gas mixture containing CO₂, after the value decrease in digestate pH from 0.1 to 1 pH point, preferably from 0.2 to 0.4 pH points, and/or a step of activating the injection of the gas mixture containing CO₂, after the increase in the value of digestate pH from 0.1 to 1 pH point, preferably from 0.2 to 0.4 pH points.

In embodiments, the apparatus of the invention further comprises a control and/or measurement system of the digestate pH, comprising a system that allows the automatic activation and/or interruption of CO₂ insufflation upon reaching preset threshold values and/or preset variations of said pH values.

### Experimental section

Some experimental tests were made by injecting CO₂ into a laboratory pilot-scale digester according to the process of the present invention and comparing the bio-methane production of such a digester with what was produced by a similar digester into which no CO₂ was injected, hereafter referred to as a conventional digester.

The process according to the present invention evaluated in the experiment involves the injection of CO₂ in the form of microbubbles with an average diameter of about 500 µm at a pressure of about 1 barg. The injection of microbubbles was performed discontinuously; in particular, injection of CO₂ was stopped when the pH value, read by the instrument placed in line with the gas supply, fell below pH 7 and was instead resumed upon reaching pH values equal to 7.5.

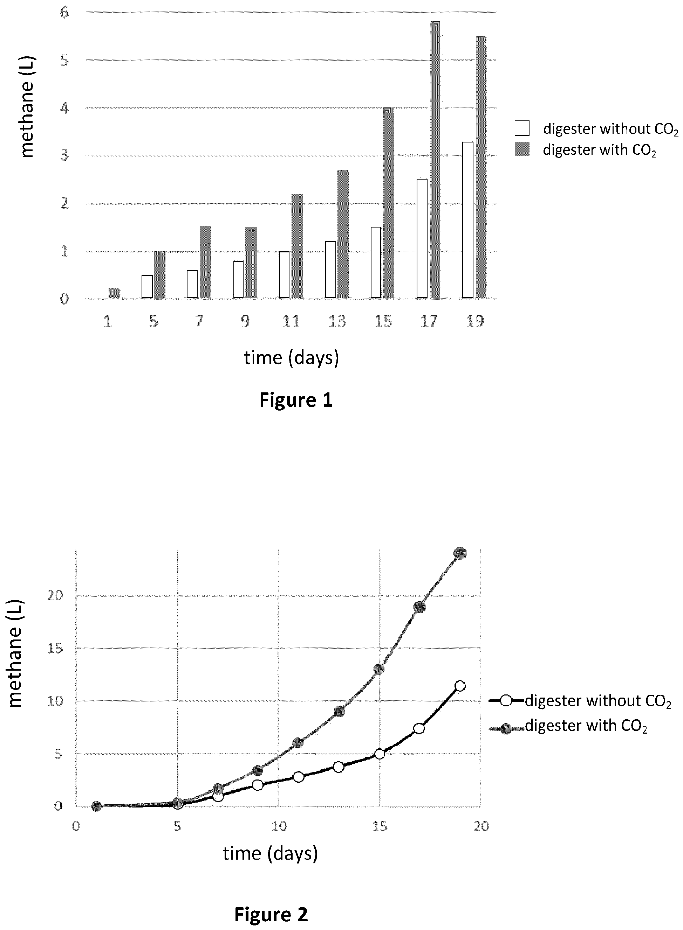

Figure 1, which shows the daily production values of methane, shows that the digester equipped with the CO₂ injection system according to the process of the present invention produces methane faster than the conventional digester, all other conditions being equal (temperature about 35°C, 5 - 6% wt/vol suspended solids, good biomass mixing). Injection of carbon dioxide according to the process of the present invention also helps to remove methane in the digester headspace. Once the daily injection process is completed, the equilibrium partial pressure of methane in the digester headspace is significantly reduced. Thus, the methane level increases as the methane produced by the bacteria is transferred to the headspace until the next injection event.

The results, graphically depicted in Figures 1 and 2, show that methane production in the digester with carbon dioxide exceeded the amount produced by the digester without CO₂ by 109% (Figure 2: cumulative production curve of methane).

The high interfacial areas, resulting from the small size of the microbubbles, and the low solubility of methane are parameters that play an important role in this process.

These factors allow the injection system to remove a large amount of methane in a short period of time, whereas the small effect on the pH value is quickly offset by the supply of alkalinity from the feed fed into and controlled by the system.

## Claims

1. A process for the production of biogas from wet anaerobic digestion plants, **characterized by** injecting a gas mixture, containing CO₂, in the form of bubbles into the digester.

2. The process according to claim 1, **characterized in that** said gas mixture comprises, in addition to CO₂, also other gases selected from methane, nitrogen and/or hydrogen, alone or in a mixture thereof.

3. The process according to claim 1, **characterized in that** said gas mixture comprises only CO₂.

4. The process according to any one of the preceding claims, **characterized in that** said bubbles have an average diameter from 50 µm to 2,000 µm, preferably from 100 µm to 1,000 µm.

5. The process according to any one of the preceding claims, **characterized in that** said CO₂, contained in said gas mixture, is that produced by the anaerobic digestion process itself and recovered from the biogas leaving the digester, after separation from the methane fraction.

6. The process according to any one of the preceding claims, **characterized in that** said gas mixture containing CO₂ is injected into the digester at a pressure from 0.5 bar to 5 bar, preferably from 0.8 bar to 3 bar, still more preferably 2 bar.

7. An apparatus (1) for anaerobic digestion of biomass, comprising:
- at least one digestate recirculation circuit (10), having at least one digestate suction branch (11) and at least one digestate return branch (13);
- at least one microbubble generating device (14);
wherein said microbubble generating device (14) has a channel (4) placed in fluidic connection with said recirculation circuit (10).

8. The apparatus according to claim 7, wherein said channel (4) is provided with a plurality of microholes (15).

9. The apparatus according to claim 7 or 8, wherein said channel (4) has a longitudinal axis (X) coaxial to said recirculation circuit (10).

10. The apparatus according to any one of claims 7-9, wherein said channel (4) has a toroidal or hollow cylindrical conformation.

11. Use of the apparatus according to any one of claims 7-10 for the process according to claim 1.

12. A control and/or measurement system of the digestate pH during the process according to any one of claims 1 to 6, said system being equipped with a system allowing the automatic activation and/or interruption of injecting said gas mixture, containing CO₂, upon reaching preset threshold values and/or preset variations of said pH values.

13. The system according to claim 12, wherein said system allows CO₂ injection to be interrupted after the value of digestate pH lowered from 0.1 to 1 pH point, preferably from 0.2 to 0.4 pH points, and activating injecting said gas mixture, containing CO₂, after the growth of the value of digestate pH from 0.1 to 1 pH point, preferably from 0.2 to 0.4 pH points.
